# EUROPÄISCHE PATENTSCHRIFT

(11) **EP 2 156 805 B1**
(45) Veröffentlichungstag und Bekanntmachung des Hinweises auf die Patenterteilung: **14.11.2012**
(21) Anmeldenummer: 08162678.0
(22) Anmeldetag: 20.08.2008
(51) Int. Cl.: A61B 19/00

(54) **Planungsunterstützung für die Korrektur von Gelenkelementen**
Planning support for correcting joint elements
Soutien de planification pour la correction d'éléments d'articulation

(43) Veröffentlichungstag der Anmeldung: 24.02.2010
(73) Patentinhaber: BrainLAB AG, 85622 Feldkirchen (DE)
(72) Erfinder: Haimerl, Martin, 82205 Gilching (DE)
(74) Vertreter: Schwabe - Sandmair - Marx

(56) Entgegenhaltungen:
- EP-A- 1 800 616
- US-B1- 6 205 411
- US-B1- 6 711 432

## Beschreibung

Die Erfindung betrifft ein Computerprogramm zur Planungsunterstützung für die Korrektur von Gelenkelementen bzw. Gelenkknochen. Gelenkknochen-Formveränderungen sind eine mögliche Ursache für arthritische Gelenkerkrankungen. Beispielsweise können im Bereich des Hüftgelenks Knochenanomalien auftreten, die bei der Beinbewegung zu Knochen-Kollisionen im Gelenkbereich führen und - insbesondere auf längere Dauer - Abnutzungserscheinungen hervorrufen. Bei den chirurgischen Korrekturmaßnahmen werden beispielsweise der Femur-Kopf oder Gelenkpfannen-Rand (Acetabulum-Rand) - beides sind Beispiele für Gelenkelemente in der Terminologie der vorliegenden Erfindung - wieder in ihre natürliche Form zurück überführt, um Kollisionen zwischen Femur und Acetabulum im Gelenkbereich zu verhindern. Dabei soll die natürliche Form des Femur-Kopfes wieder hergestellt werden.

Bei offenen (nicht-arthroskopischen) Eingriffen werden gemäß dem Stand der Technik manchmal kugelförmige Schablonen verwendet, um die Sphärizität bzw. Kugelform des Femur-Kopfes zu überprüfen. Mit solchen und anderen derzeit verwendeten Mitteln ist ein systematischer Abgleich zwischen dem gewünschten Zustand und der momentanen Gelenkelement-Form nicht akkurat möglich. Insbesondere ist die Überwachung und Steuerung der Abrasionstiefe nicht mit der notwendigen und gewünschten Genauigkeit realisierbar.

Die US 6,205,411, die EP 1 800 616 und die US 2007/0233269 befassen sich mit dem Setzen von Gelenkimplantaten.

Es ist deshalb eine Aufgabe der vorliegenden Erfindung, ein Computerprogramm zur Planungsunterstützung für die Korrektur von Gelenkelementen bereitzustellen, welches die Korrektur und Wiederherstellung von Gelenkelementen optimiert. Insbesondere soll eine Navigationsunterstützung, d.h. eine Planungsunterstützung bereitgestellt werden, welche es möglich macht, die Abrasionstiefe zu navigieren.

Diese Aufgabe wird durch ein Computerprogramm zur Planungsunterstützungsverfahren gemäß dem Anspruch 1 gelöst. Die Unteransprüche definieren bevorzugte Ausführungsformen der Erfindung.

Allgemein ausgedrückt besteht die Erfindung also in einem Computerprogramm zur Planunasunterstützung für die Rücküberführung von Gelenkelementen in ihre natürliche Soll-Form das, wenn es auf einem Computer eines Navigationssystems mit einem navigierten Instrument läuft oder in einen solchen Computer geladen ist, den Computer veranlasst,
- die mittels des navigierten Instruments erfassten Daben über die Ist-Form mindestens eines interessierenden Teils des Gelenkelements (17) intraoperativ einzulesen bzw. zu speichern;
- die Ist-Form mit der Soll-Form zu vergleichen; und
- die Vergleichsdaten, insbesondere die Abweichungen zwischen Ist- und Soll-Form, zur Planung der Tiete der Abrasion von abzutragenden Gelenkelementteilen (17) anzuzeigen.

Durch den Formvergleich und die Einbringung von dessen Ergebnis in die computergestützte Planung werden die hohen Genauigkeitsanforderungen erfüllt, die notwendig sind um die Abrasionstiefe zu navigieren. Die Erfindung liefert also eine spezielle Technik, um die Ist-Form während der Abrasionsprozedur zu überprüfen, und sie stellt damit eine Navigation bereit, welche die Konturtreue, beispielsweise die Sphärizität des Femur-Kopfes innerhalb des Gelenkbereiches überprüfen kann. Mit dieser Überprüfung kann dann die korrekte Abrasionstiefe richtig eingeschätzt werden. Eine gute postoperative Form ist für den längerfristigen Erfolg des Eingriffs notwendig, und deshalb liefert eine genaue Abrasions-Navigation gemäß der vorliegenden Erfindung einen großen Vorteil für einen Chirurgen, speziell auch für Chirurgen mit geringem Erfahrungsschatz.

Mit anderen Worten ausgedrückt liefert die Erfindung eine intraoperative Möglichkeit zur Konturenüberprüfung an zu bearbeitenden Gelenkelement-Oberflächen. Sie geht damit über die herkömmliche Navigation hinaus, die lediglich vorab akquirierte Daten (z.B. MR- oder CT-Daten) als Basis für die gesamte Operation nutzt.

Bei einer Ausführungsform der vorliegenden Erfindung wird die Ist-Form mittels eines Instruments erfasst bzw. abgegriffen, das mit Hilfe eines medizintechnischen Navigation- und Trackingsystems positionell bestimmt werden kann. Insbesondere kann es sich dabei um einen navigierten und getrackten Pointer handeln.

Die Ist-Form kann ganz allgemein zweidimensional oder dreidimensional durch ein solches navigiertes Instrument erfasst werden. Ein weiteres Beispiel hierfür ist eine dreidimensionale Rekonstruktion der Gelenkelement-Oberfläche, wobei letztere mit mindestens einem Bilderfassungsgerät erfasst wird, insbesondere mit mindestens einer Endoskopkamera. Dabei kann die Ist-Form durch eine oder mehrere der folgenden Methoden ermittelt werden:
- Formermittlung mittels Schattierungsanalyse;
- Formermittlung mit Hilfe mehrerer Bilder, die während einer Relativbewegung zwischen Bilderfassungsgerät und Gelenkelementoberfläche erfasst werden;
- Formermittlung mittels stereoskopischer Bilder (zwei Kameras);
- Formermittlung mittels Texturbestimmung, insbesondere an Abrasionsflächen bzw. schon der Abrasion unterzogenen Flächen.

Eine weitere Ausführungsvariante ermittelt die Ist-Form unter Verwendung spezieller geometrischer Eigenschaften, ohne eine gesamte dreidimensionale Formrekonstruktion auszuführen, und zwar aus Navigationsdaten wie akquirierten Punkten oder Endoskopbildern. Dabei werden speziell Oberflächennormalen oder die Krümmung an unterschiedlichen Teilen der Gelenkelement-Oberfläche rekonstruiert. Eine weitere Möglichkeit der Bestimmung der Ist-Form besteht in der Verwendung von intraoperativ einsetzbaren Bildakquirierungsgeräten, speziell Ultraschallsonden oder Fernabtastern speziell Laserabtastern. Alle möglichen berührungslosen Abtasttechniken können verwendet werden.

Es besteht ferner die Möglichkeit, die Ist-Form unter Verwendung von navigierten bzw. getrackten Abgreifern zu ermitteln, welche die Gelenkoberfläche an mehr als einem Punkt abtasten, insbesondere an drei Punkten. Die Abtastung kann grundsätzlich auch linienförmig oder flächig (z.B. an verschiedenen Teilflächen) stattfinden. Solche Abgreifer sind bei einer Ausführungsform Schablonen, speziell navigierte bzw. getrackte Krümmungsschablonen, Kugelformschablonen oder Weichschablonen, die einen negativen Abdruck der Gelenkknochenoberfläche erzeugen.

Die Soll-Form wird im Rahmen der Erfindung bevorzugt mittels vorbekannter Informationen festgelegt, und insbesondere aus
- akquirierten Patientendaten;
- Modelldaten, Zielformen oder Formvorgaben für das Gelenkelement;
- generischen Formen, wie angenäherte Kugelformen;
oder aus einer Kombination aus zwei oder mehreren dieser Informationen.

Die Erfindung betrifft ferner ein Computerprogramm-Speichermedium, das ein solches Programm aufweist.

Die Erfindung wird im Weiteren anhand von Ausführungsformen und mit Hilfe der beiliegenden Zeichnungen näher erläutert. Sie kann alle hierin beschriebenen Merkmale einzeln sowie in jedweder Kombination umfassen. Insbesondere kann die Erfindung auch als Vorrichtung oder System von Einzelvorrichtungen aufgefasst werden, wobei die Vorrichtungen bzw. die Vorrichtungsteile dann die Funktionen erfüllen, welche hierin verfahrensmäßig beschrieben werden, und die Kombination der Vorrichtung bzw. Vorrichtungsteile dann die entsprechende Erfindung ausmacht. Entsprechendes gilt für die Verwendung solcher Vorrichtungen bzw. Vorrichtungsteile und spezieller Verfahrensschritte im Rahmen erfindungsgemäßer Anwendungen. In den Zeichnungen zeigen:
- Figur 1: ein Navigations-Umfeld für die erfindungsgemäße Planungsunterstützung; und
- Figur 2: Beispiele für Bilddarstellungen im Rahmen einer erfindungsgemäßen Navigation bzw. Planungsunterstützung.

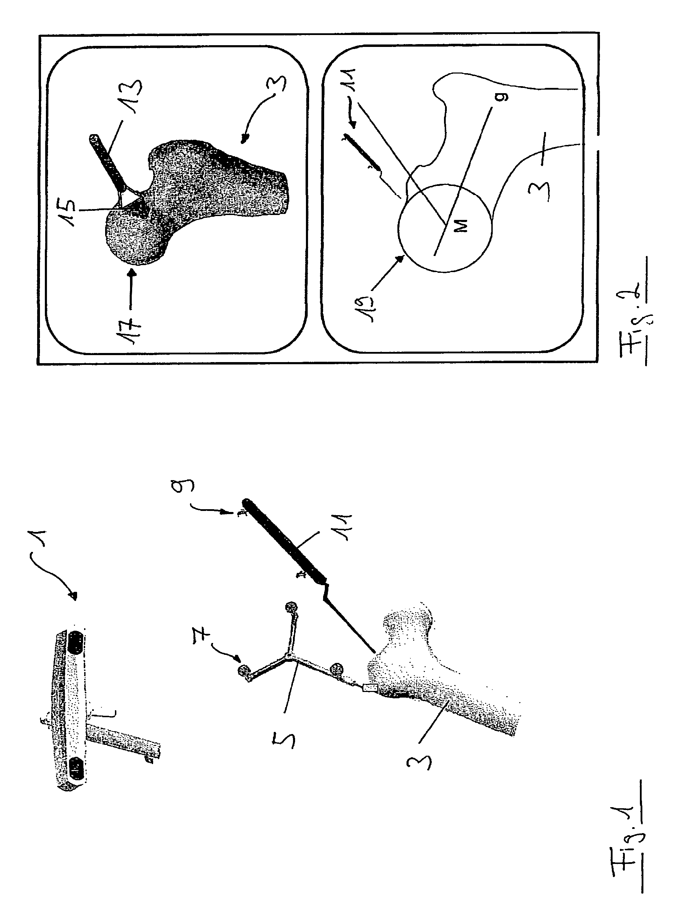

Bei einer Ausführungsform der Erfindung wird die momentane Knochenstruktur (während des Abrasionsverfahrens) durch einen Pointer durchgeführt, sowie durch den Vergleich der erhaltenen Form mit der gewünschten Knochenform. Eine Rekonstruktion der momentanen Knochenform sollte dabei durchgeführt werden, und diese Rekonstruktion kann durch bekannte Informationen unterstützt werden (z.B. gewünschte Form, Modellkenntnisse oder generische Form wie z.B. angenäherte Kugel). In der Figur 1 ist das Navigationsumfeld gezeigt, welches für diese Ausführungsform verwendet wird. Das Navigationssystem weist eine Kameraanordnung 1, die Navigationshardware 2 (nur schematisch dargestellt) und eine Referenzanordnung 5 auf, die starr mit dem Oberschenkelknochen (Femur) 3 verbunden ist. An der Referenzanordnung 5 befindet sich drei passive Kugelmarker 7, die vom Kamerasystem 1 erfasst werden, so dass die Raumlage des Knochens 3 über die Referenzanordnung 5 mit dem Navigationssystem bestimmt werden kann. In der Figur 1 ist auch ein Pointer 11, also ein medizinisches Zeigegerät zu sehen, das ebenfalls über die Navigation positionell verfolgt d.h. getrackt wird. Hierzu weist der Pointer 11 die beiden Kugelmarker 9 auf. Durch Vorabkalibrierung oder durch die Verwendung eines vorkalibrierten Instruments kann die Position der Spitze des Pointers 11 jederzeit festgestellt werden.

Dieser letztere Umstand kann nun dazu verwendet werden, während einer Abrasions-Operation die tatsächliche Form eines Femur-Kopfes zu erfassen. Mit Hilfe der so er Daten kann dann im Navigationssystem die momentane Knochenform rekonstruiert werden und anhand dieser momentanen Knochenform kann die notwendige Abrasionstiefe an jeder Stelle des Gelenkknochens immer zeitnah festgestellt werden, so dass eine Abrasion sicher und auch nur an den notwendigen Stellen erfolgen wird und der Operationserfolg in hohem Maße garantiert wird.

Das Erfassen der Ist-Knochenform, die Rekonstruktion des Knochens und die Navigation der Abrasionstiefe werden nun anhand der Darstellung der Figur 2 etwas näher erläutert. Die Figur 2 zeigt beispielhafte Bildschirmdarstellungen eines Navigationssystems (Ausgabe am Navigationsbildschirm 4).

Bei einer Ausführungsform der Erfindung wird eine 3D-Rekonstruktion der Knochenoberfläche mit Hilfe berührungsloser Erfassung, beispielsweise durch Endoskopbilder, Laserabtaster, Ultraschallsonden, durchgeführt. Insbesondere bei der endoskopischen Bilderfassung kann die Form aus der Schattierung, aus Bewegungsbildern und Stereobildern oder aus Texturen abgeleitet werden. Speziell eignet sich für eine Rekonstruktion der Form die Texturerfassungsmethode, weil der Knochen bei Abrasion also in den Abrasionsgebieten eine gewisse Textur verliehen bekommt. Diese Textur entsteht durch den Abrasionsprozess selbst und die innere Struktur des Knochens. Modellinformationen können zusätzlich verwendet werden. Die obere Darstellung in der Figur 2 zeigt beispielsweise eine solche berührungslose Erfassung eines Gebiets 15 auf dem Femur-Kopf 17 des Femurs 3. Die Vorrichtung 13 kann hier eine Endoskopkamera, aber auch jedes andere berührungslose Abtastgerät sein, und sie erfasst gerade mit ihrem Tastbereich (Taststrahl) 14 einen Teil des Bereichs 15 am Femur-Kopf 17.

Die untere Darstellung der Anzeige 4 in Figur 2 zeigt auf, wie Modelldaten bzw. generische einbeschriebene Formen erfindungsgemäß verwendet werden können. Der Pointer 11 wird in seiner tatsächlichen Stellung eingeblendet, bei der er mit seiner Spitze auf einem Knochenüberstand am Femur-Kopf sitzt. Die gewünschte Form des Femur-Kopfes ist als Kugelform 19 in der Navigation eingeblendet und rekonstruiert, wobei die Neckachse g und der darauf sitzende Mittelpunkt M für die Kugeldarstellung und -positionierung verwendet werden. Im vorliegenden Fall wird das System anzeigen, dass die Spitze des Pointers 11 sich noch um einen gewissen Abstand über der Kreislinie 19 befindet, beispielsweise etwa 1,5 mm, und aus dieser Information folgt unmittelbar die momentane Abrasionstiefe an der mit der Spitze angefahrenen Stelle.

Schließlich soll noch angemerkt werden, dass andere Ausführungsformen möglich sind, welche die Grundsätze der erfindungsgemäßen Formerfassung berücksichtigen aber Spezialvorrichtungen zum Akquirieren von Daten über die Knochenoberfläche verwenden. Diese Vorrichtungen können sein:
- Spezialvorrichtungen für die intraoperative Bilddatenakquirierung (z.B. Ultraschall, Laser, intraoperatives CT, intraoperatives MR);
- robotisch unterstützte Akquirierung von Oberflächenpunkten, wobei der Roboter oder ein Tele-Manipulator als Navigationsvorrichtung verwendet werden können. Das System kann an dem Knochen fixiert sein (z.B. durch eine spezielle Klammerart), und die verbleibende (relative) Bewegung muss aufgezeichnet werden. Dies sollte die (relative) Genauigkeit erhöhen;
- Spezial-Hardwarevorrichtungen zur Akquirierung von Informationen über die Sphärizität/Krümmung der Knochenoberfläche an einem bestimmten Punkt. Beispielsweise können getrackte bzw. navigierte, kugelförmige Schablonen oder Templates oder Pointer mit spezifischen geometrischen Strukturen verwendet werden. Auch Soft-Templates, die einen negativen Abdruck der Knochenoberfläche erzeugen, sind einsetzbar. Ein weiteres Beispiel für eine Spezial-Hardwarevorrichtung wäre ein Pointer mit mindestens drei Spitzen zur Akquirierung der Oberflächenausrichtung an einer bestimmten Position. Die drei Spitzen müssen während der Akquirierung an der Oberfläche positioniert sein und deren Ausrichtung kann dann aus der Ausrichtung des Pointer-Griffes bestimmt werden.

## Patentansprüche

1. Computerprogramm zur Planungsunterstützung für die Rücküberführung von Gelenkelementen in ihre natürliche Soll-Form das, wenn es auf einem Computer eines Navigationssystems mit einem navigierten Instrument läuft oder in einem solchen Computer geladen ist, den Computer veranlasst,
- die mittels des navigierten Instruments erfassten Daten über die Ist-Form mindestens eines interessierenden Teils des Gelenkelements (17) intraoperativ einzulesen bzw. zu speichern:
- die Ist-Form mit der Soll-Form zu vergleichen; und
- die Vergleichsdaten, nämlich die Abweichungen zwischen Ist- und Soll-Form, zur Planung der Tiefe der Abrasion von abzutragenden Gelenkelementteilen (17) anzuzeigen.

2. Programm nach Anspruch 1, bei dem die Ist-Form mittels des Instruments (9, 13) insbesondere mittels eines navigierten und getrackten Pointers (11) erfasst bzw. abgegriffen wird, das mit Hilfe eines medizintechnischen Navigations- und Trackingsystems (1, 2) positionell bestimmt wird.

3. Programm nach Anspruch 1 oder 2, bei dem das Instrument ein Bilderfassungsgerät ist und die Ist-Form mittels einer Rekonstruktion, speziell einer dreidimensionalen Rekonstruktion der Gelenkelementoberfläche erfasst wird, die bildlich mit mindestens dem Bilderfassungsgerät erfasst wird, insbesondere mit mindestens einer Endoskopkamera (13).

4. Programm nach Anspruch 3, bei dem die Ist-Form bei der Rekonstruktion durch eine oder mehrere der folgenden Methoden ermittelt wird:
- Formermittlung mittels Schattierungsanalyse;
- Formermittlung mit Hilfe mehrerer Bilder, die während einer Relativbewegung zwischen Bilderfassungsgerät und Gelenkelementoberfläche erfasst werden;
- Formermittlung mittels stereoskopischer Bilder;
- Formermittlung mittels Texturbestimmung, insbesondere an Abrasionsflächen bzw. schon der Abrasion unterzogenen Flächen.

5. Programm nach Anspruch 1 oder 2, bei dem die Ist-Form mit dem Instrument unter Verwendung spezieller geometrischer Eigenschaften ermittelt wird, ohne eine gesamte dreidimensionale Formrekonstruktion auszuführen, und zwar aus Navigationsdaten, wie akquirierten Punkten oder Endoskopbildern.

6. Verfahren Programm nach Anspruch 5, bei dem Oberflächennormalen oder die Krümmung an unterschiedlichen Teilen der Gelenkelementoberfläche rekonstruiert werden.

7. Programm nach Anspruch oder 2, bei dem das Instrument ein intraoperativ einsetzbares Bildaquirierungsgerät ist und, die Ist-Form unter Verwendung des intraoperativ einsetzbaren Bildakquirierungsgerätes, speziell einer Ultraschallsonde, oder eines Fernababtasters, speziell Laserabtasters, ermittelt wird.

8. Programm nach Anspruch 1 oder 2, bei dem die Ist-Form mit dem Instrument unter Verwendung einer robotisch oder telemanipulatorisch unterstützten Akquirierung von Oberflächenpunkten ermittelt wird.

9. Programm nach Anspruch 1 oder 2, bei dem das Instrument ein navigierter bzw. getrackten Abgreifer ist und die Ist-Form unter Verwendung des navigierten bzw. getrackten Abgreifers ermittelt wird, welcher die Gelenkoberfläche an mehr als einem Punkt abtastet, insbesondere an drei Punkten, linienförmig oder flächig.

10. Programm nach Anspruch 9, bei dem die Abgreifer Schablonen sind, speziell navigierte bzw. getrackte Krümmungsschablonen, Kugelformschablonen oder Weichschablonen, die einen negativen Abdruck der Gelenkknochenoberfläche erzeugen.

11. Programm nach einem der Ansprüche 1 bis 10, bei dem die Soll-Form mittels vorbekannter Informationen festgelegt wird, insbesondere aus
- akquirierten Patientendaten;
- Modelldaten, Zielformen oder Formvorgaben für das Gelenkelement;
- generischen Formen, wie angenäherte Kugelformen;
oder aus einer Kombination aus zwei oder mehreren dieser Informationen.

12. Computerprogramm-Speichermedium, das ein Programm gemäß einem der Ansprüche 1 bis 11 aufweist.

## Claims

1. A computer program for assisting in planning for returning joint elements to their intended, natural shape which, when it is running on a computer of a navigation system comprising a navigated instrument or when it is loaded onto such a computer, causes the computer to:
- intra-operatively read in and/or store the data, captured by means of the navigated instrument, on the actual shape of at least one part of the joint element (17) which is of interest;
- compare the actual shape with the intended shape; and
- indicate the comparison data, i.e. the deviations between the actual shape and the intended shape, in order to plan the abrasion depth of joint element parts (17) which are to be ablated.

2. The program according to claim 1, wherein the actual shape is detected and/or acquired by means of the instrument (9, 13), in particular by means of a navigated and tracked pointer (11), which is positionally determined with the aid of a medical navigation and tracking system (1, 2).

3. The program according to claim 1 or 2, wherein the instrument is an image detection device and the actual shape is detected by means of a reconstruction, specifically a three-dimensional reconstruction, of the joint element surface, which is visually detected using at least the image detection device, in particular using at least one endoscopic camera (13).

4. The program according to claim 3, wherein the actual shape is ascertained by reconstruction using one or more of the following methods:
- ascertaining the shape by means of a shading analysis;
- ascertaining the shape with the aid of a number of images which are captured during a relative motion between the image detection device and the joint element surface;
- ascertaining the shape by means of stereoscopic images;
- ascertaining the shape by determining the texture, in particular on abrasion areas and/or areas which have already been subjected to abrasion.

5. The program according to claim 1 or 2, wherein the actual shape is ascertained using the instrument and specific geometric properties, without completely reconstructing a three-dimensional shape, namely from navigation data such as acquired points or endoscopic images.

6. The program according to claim 5, wherein surface normals or the curvature on different parts of the joint element surface are reconstructed.

7. The program according to claim 1 or 2, wherein the instrument is an image acquisition device which can be used intra-operatively and the actual shape is ascertained using the image acquisition device which can be used intra-operatively, specifically an ultrasound probe or remote scanner, specifically a laser scanner.

8. The program according to claim 1 or 2, wherein the actual shape is ascertained using the instrument and robot-assisted or telemanipulator-assisted acquisition of surface points.

9. The program according to claim 1 or 2, wherein the instrument is a navigated and/or tracked pointer and the actual shape is ascertained using the navigated and/or tracked pointer which scans the joint surface at more than one point, in particular at three points, linearly or over an area.

10. The program according to claim 9, wherein the pointers are templates, specifically navigated and/or tracked curvature templates, spherical templates or soft templates which generate a negative imprint of the joint bone surface.

11. The program according to any one of claims 1 to 10, wherein the intended shape is determined by means of prior-known information, in particular from:
- acquired patient data;
- model data, target shapes or predetermined shapes for the joint element;
- generic shapes, such as approximated spheres;
or from a combination of two or more of these types of information.

12. A computer program storage medium which comprises a program in accordance with any one of claims 1 to 11.

## Revendications

1. Programme informatique pour l'assistance de planification pour la retouche d'éléments d'articulation vers leur forme théorique naturelle, qui, lorsqu'il est exécuté sur un ordinateur d'un système de navigation avec un instrument navigué ou chargé dans un tel ordinateur, fait en sorte que l'ordinateur effectue les tâches suivantes :
- lire ou enregistrer de manière intra-opérative les données relevées à l'aide de l'instrument navigué concernant la forme réelle d'au moins une partie intéressante de l'élément d'articulation (17) ;
- comparer la forme réelle à la forme théorique et
- afficher les données comparatives, à savoir les écarts entre la forme réelle et la forme théorique, pour la planification de la profondeur d'abrasion des parties d'éléments d'articulation (17) à éroder.

2. Programme selon la revendication 1, dans lequel la forme réelle est relevée ou mesurée à l'aide de l'instrument (9, 13), notamment un pointeur (11) navigué et tracé, dont la position est déterminée à l'aide d'un système médical de navigation et de pistage (1, 2).

3. Programme selon la revendication 1 ou 2, dans lequel l'instrument est un dispositif d'enregistrement d'images et la forme réelle est relevée à l'aide d'une reconstitution, plus particulièrement d'une reconstitution tridimensionnelle de la surface de l'élément d'articulation, relevée graphiquement avec au moins le dispositif d'enregistrement d'images, notamment avec au moins une caméra endoscopique (13).

4. Programme selon la revendication 3, dans lequel la forme réelle est déterminée, lors de la reconstitution, à l'aide d'une ou plusieurs des méthodes suivantes :
- détermination de la forme par analyse des ombres ;
- détermination de la forme à l'aide de plusieurs images prises pendant le mouvement relatif entre le dispositif d'enregistrement d'images et la surface de l'élément d'articulation :
- détermination de la forme à l'aide d'images stéréoscopiques ;
- détermination de la forme à l'aide des textures, plus particulièrement sur les surfaces d'abrasion ou sur les surfaces déjà soumises à l'abrasion.

5. Programme selon la revendication 1 ou 2, dans lequel la forme réelle est déterminée avec l'instrument à l'aide de propriétés géométriques spécifiques sans effectuer de reconstitution de forme tridimensionnelle, à partir de données de navigation comme des points acquis ou des images endoscopiques.

6. Programme selon la revendication 5, dans lequel les normales de la surface ou la courbure sont reconstituées dans différentes parties de la surface de l'élément d'articulation.

7. Programme selon la revendication 1 ou 2, dans lequel l'instrument est un dispositif d'enregistrement d'images utilisable de manière intra-opérative et la forme réelle est déterminée à l'aide du dispositif d'enregistrement d'images, plus particulièrement d'une sonde à ultrasons ou d'un capteur distant, plus particulièrement de capteurs laser.

8. Programme selon la revendication 1 ou 2, dans lequel la forme réelle est déterminée avec l'instrument à l'aide d'une acquisition de points de la surface assistée par robot ou par télémanipulateur.

9. Programme selon la revendication 1 ou 2, dans lequel l'instrument est une griffe naviguée ou pistée et la forme réelle est déterminé à l'aide de la griffe naviguée ou pistée, qui palpe la surface de l'articulation sur plusieurs points, plus particulièrement sur trois points, de manière linéaire ou sur la surface.

10. Programme selon la revendication 9, dans lequel les griffes sont des gabarits, plus particulièrement des gabarits de courbure, des gabarits de forme sphérique ou des gabarits souples navigués ou pistés qui produisent une empreinte négative de la surface osseuse de l'articulation.

11. Programme selon l'une des revendications 1 à 10, dans lequel la forme théorique est déterminée à l'aide d'informations déjà connues, plus particulièrement à l'aide :
- de données acquises sur le patient ;
- de données modélisées, de formes cibles ou de modèles pour l'élément d'articulation ;
- de formes génériques, comme des formes sphériques approximatives ;
ou à l'aide d'une combinaison de deux de ces infirmations ou plus.

12. Support de stockage de programme informatique qui comprend un programme selon l'une des revendications 1 à 11.
